(19) Europäisches Patentamt
European Patent Office
Office européen des brevets

(11) Publication number: **0 257 787 B1**

# (12) EUROPEAN PATENT SPECIFICATION

(45) Date of publication of patent specification: **17.04.91**

(51) Int. Cl.5: **C07C 311/15**

(21) Application number: **87306453.9**

(22) Date of filing: **21.07.87**

Divisional application 90104584.9 filed on 21/07/87.

## (54) Process for producing optically active benzene-sulfonamide derivates.

(30) Priority: **21.07.86 JP 172285/86**

(43) Date of publication of application:
**02.03.88 Bulletin 88/09**

(45) Publication of the grant of the patent:
**17.04.91 Bulletin 91/16**

(84) Designated Contracting States:
**AT DE ES FR GB IT**

(56) References cited:
**EP-A- 0 034 432**
**EP-A- 0 092 787**
**US-A- 4 000 197**

(73) Proprietor: **YAMANOUCHI PHARMACEUTICAL CO. LTD.**
**No. 3-11 Nihonbashi-Honcho, 2-chome Chuo-ku**
**Tokyo(JP)**

(72) Inventor: **Okada, Minoru**
**10-20 Higashi Oi 6-chome Shinagawa-ku**
**Tokyo 140(JP)**
Inventor: **Yoshida, Koichi**
**9-16, Shirokane 3-chome Minato-ku**
**Tokyo 108(JP)**
Inventor: **Takanobu, Kiyoshi**
**1142-1, Hongo-cho**
**Omiya-shi Saitama 330(JP)**

(74) Representative: **Geering, Keith Edwin et al**
**REDDIE & GROSE 16 Theobalds Road**
**London WC1X 8PL(GB)**

EP 0 257 787 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a new process for producing optically active benzenesulfonamide derivatives which are represented by the following general formula (I) and are useful as antihypotensive agents and as intermediates for the manufacture of optically active drugs,

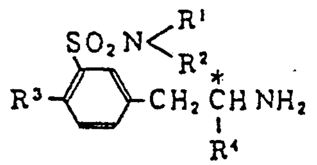

(I)

wherein $R^1$ and $R^2$ are the same or different and selected independently from a hydrogen atom and lower alkyl groups; $R^3$ is a hydrogen atom or a lower alkyl, hydroxyl or lower alkoxyl group; and $R^4$ stands for a lower alkyl group.

Most of the compounds (I) are known from Japanese Patent Publication No.18353 (1983) which discloses compounds of formula (A-I)

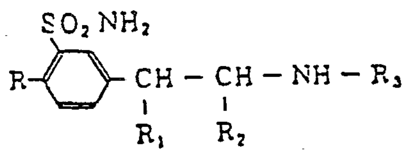

( A − I )

(wherein R is a lower alkyl, lower alkoxyl or hydroxyl group; $R_1$ is a hydrogen or halogen atom or hydroxyl; $R_2$ is a hydrogen atom or a lower alkyl group; and $R_3$ is a hydrogen atom or a lower alkyl group), indicates that all the related optical isomers are included, and states that the compounds (A-I) are useful as antihypotensive agents with no side effects, such as increased heart rate and palpitation.

We disclosed in Japanese Patent publication No.110665 (1981), particularly in Examples 23 and 24, the sulfamoyl-substituted phenetylamine derivatives having adrenergic receptor α-blocking effect and useful as hypotensive agents can be prepared from the optically active compounds

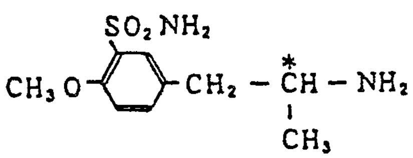

[A-I-a-free, R(-) or S(+)]

Manufacture of these optically active starting materials was not described, but they were in fact prepared by optical resolution of a racemic mixture [a mixture of A-I-a, R(-) and S(+)] obtained by the method described in Japanese Patent publication No.18353 (1983) mentioned above via reaction steps given below:

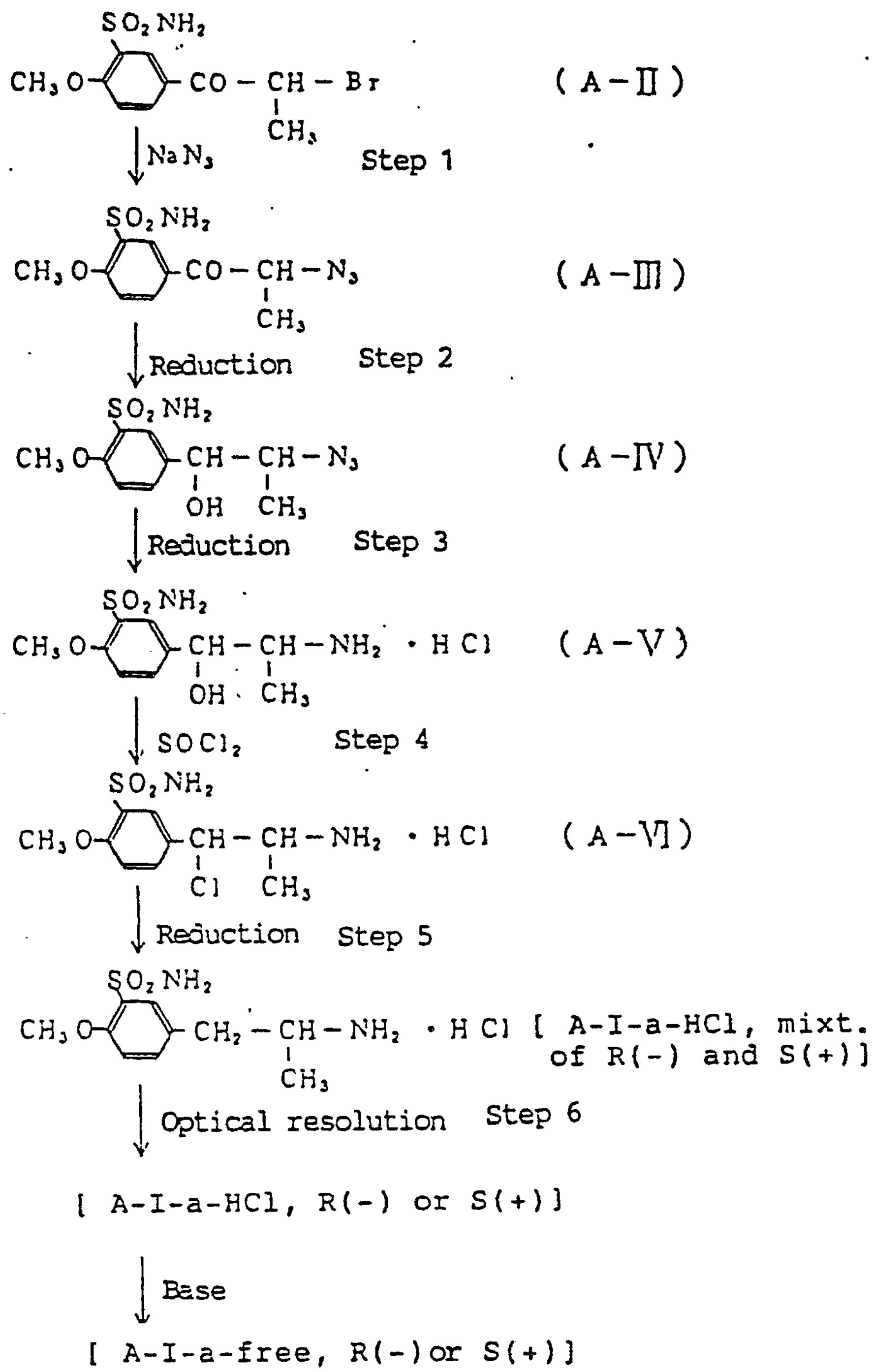


( A-II )

( A-III )

( A-IV )

( A-V )

( A-VI )

[ A-I-a-HCl, mixt. of R(-) and S(+)]

[ A-I-a-HCl, R(-) or S(+)]

[ A-I-a-free, R(-) or S(+)]

In the prior manufacturing method, salts of compounds (I) are prepared from haloalkyl sulfamoylphenyl ketone (II) via six reaction steps; this method is cumbersome because of the many reaction steps involved and the low product yield.

When only one optical isomer of a compound (I) is put to use as a drug, the useful yield will be less than half, leading to a further increase in the manufacturing cost.

This disadvantage is more marked when a compound (I) is used as an intermediate for the manufacture of optically active drugs; its practical use is not advantageous, as with another type of intermediate described in Japanese Patent publication No.10665, m-(1-halo-2-substituted-aminoalkyl)-0-substituted-benzenesul-fonamides, whose optical resolution is difficult.

Intensive studies in search for a more advantageous method for synthesizing the compounds (I) have led us to find that these can be simply prepared with unusually high reaction and optical yields by diastereoisomeric separation.

Thus this invention provides a process for producing compounds of formula (I), which comprises

decomposing a m-(2-substituted-alkylaminoalkyl)benzenesulfonamide derivative of formula (II)

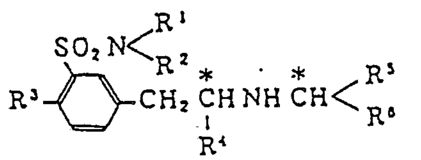

(II)

(wherein $R^1$, $R^2$, $R^3$ and $R^4$ are defined above; $R^5$ denotes a lower alkyl, a carboxy-lower-alkyl or a lower-alkoxycarbonyl-lower-alkyl group; and $R^6$ is a carboxyl or a lower-alkoxycarbonyl group. Embodiments wherein $R^1$-$R^5$ are as defined above and $R^6$ is a substituted or unsubstituted phenyl group are the subject of a divisional application.

The compounds (II) can best be prepared by reaction of sulfamoyl-substituted benzyl lower alkyl ketone (III)

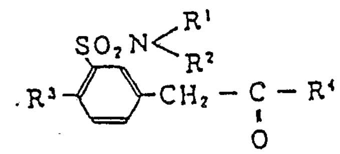

(III)

(wherein $R^1$, $R^2$, $R^3$ and $R^4$ are as defined above), with optically active, substituted alkylamine (IV)

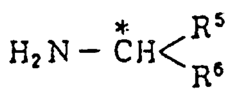

(IV)

(wherein $R^5$ and $R^6$ are as defined above), in the presence of reducing agent. In the definition of groups herein, the term "lower" means, unless otherwise specified, linear or branched chains of 1 to 5 carbon atoms, preferably those containing no asymmetric carbon.

Thus, illustrative examples of lower alkyl groups include methyl, ethyl, propyl, isopropyl, butyl, isobutyl, tert-butyl, pentyl,isopentyl and neopentyl groups; and those of lower alkoxyl groups include methoxy, ethoxy, propoxy, isopropoxy, butoxy, isobutoxy, tert-butoxy, pentyloxy and isopentyloxy groups. Particularly, the lower alkyl represented by $R^5$ is preferably methyl, ethyl, isopropyl or isobutyl, most preferably methyl.

Typical examples of carboxy-lower-alkyl groups include carboxymethyl, carboxyethyl, carboxypropyl, carboxybutyl and carboxypentyl groups. As examples of lower alkoxycarbonyl-lower-alkyl groups, may be mentioned esters of the above carboxy-lower-alkyls with a lower alkanol, such as methoxycarbonylmethyl, ethoxycarbonylmethyl, propoxycarbonylmethyl, isopropoxycarbonylmethyl, butoxycarbonylmethyl, isobutoxycarbonylmethyl, tert-butoxycarbonylmethyl, pentyloxycarbonylmethyl, methoxycarbonylethyl, ethoxycarbonylethyl, propoxycarbonylethyl, isopropoxycarbonylethyl, butoxycarbonylethyl, isobutoxycarbonylethyl, tert-butoxycarbonylethyl, pentyloxycarbonylethyl, methoxycarbonylpropyl, ethoxycarbonylpropyl, tert-butoxycarbonylpropyl, methoxycarbonylbutyl, ethoxycarbonylbutyl, tert-butoxycarbonylbutyl, methoxycarbonylpentyl, ethoxycarbonylpentyl and tertbutoxycarbonylpentyl.

"Decomposition" herein means a decomposition reaction in which $R^5$-$CH_2$-$R^6$ or $R^5$-CO-$R^6$ is stereospecifically eliminated from a compound (II); the method includes the formation of a Schiff base

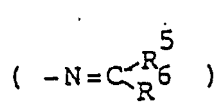

followed by hydrolysis.

When $R^6$ is a carboxyl or a lower-alkoxycarbonyl group, the compound (IV) is an optically active amino

acid or a lower alkyl ester thereof. Preferred examples of such amino acids include alanine, valine, aspartic acid and glutamic acid.

Shown below is the flow chart for the manufacturing process of this invention.

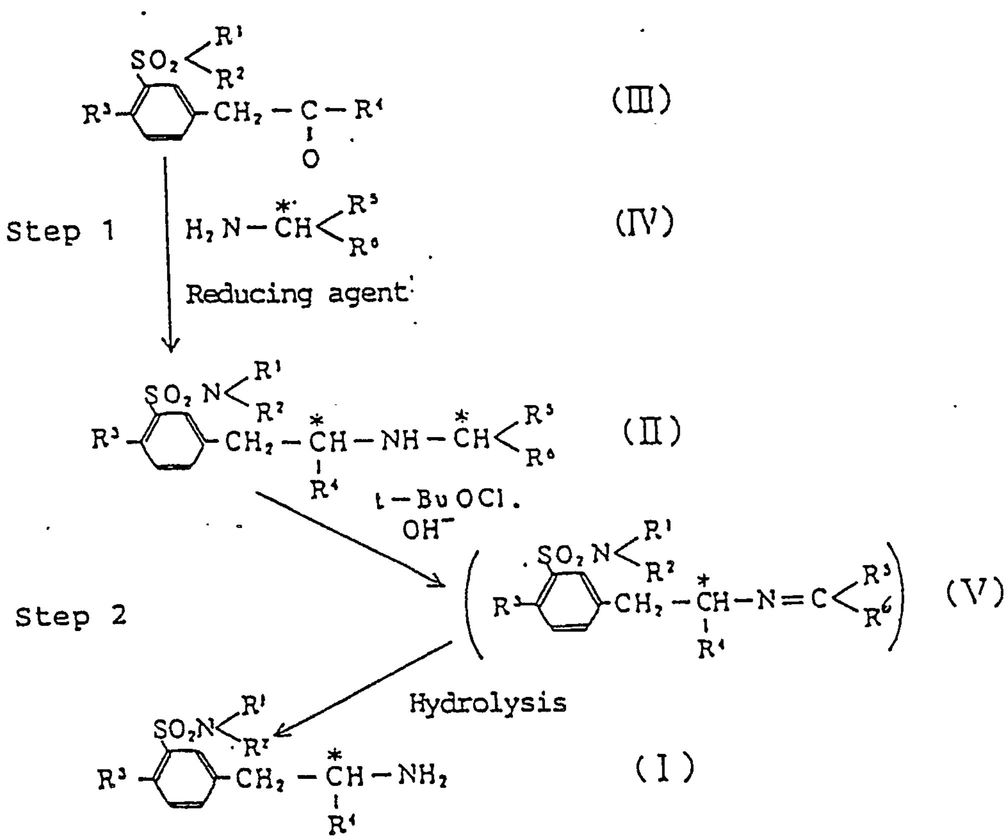


(wherein $R^1$, $R^2$, $R^3$, $R^4$, $R^5$ and $R^6$ are as defined above; and $R^7$ is a hydrogen atom or a lower-alkoxycarbonyl group).

Preferred details for each reaction step in the process of the invention are given below.

Step 1

Compound (II) is prepared by condensation between compound (III) and compound (IV) in the presence of reducing agent.

The reaction can be carried out in an organic solvent, such as methanol, ethanol or benzene, using the two reactants in nearly equimolar amounts (or with a slight excess of either) one of them), in the presence of lithium aluminum hydride, sodium cyanoborohydride, sodium borohydride or borane, or by catalytic reduction, under elevated pressure as required, in the presence of a catalyst such as Raney nickel, platinum/carbon, palladium/carbon and platinum oxide, or by electrolytic reduction using copper or platinum as cathode. Of the three modes of reduction mentioned above, catalytic reduction is the most preferred because of the ease of operation and higher optical yield.

The reaction temperature and time may vary depending on the type and quantity of reactants used, the type of reducing agent, pressure conditions and other factors. However, the reaction is generally carried out at room temperature or at elevated temperature, preferably at about 40 to 80 °C, for 1 to 48 hours under normal pressure and for a shorter time under an elevated pressure.

The pressure, if applied, is generally 1 to 100 atm, preferably 2 to 20 atm.

The above-described synthesis of secondary amines by reductive condensation between a carbonyl compound and an amine may be allowed to proceed in two separate steps: dehydration reaction ( for example, by the use of Dean-Stark trap ) to form a Schiff base, and reduction of the isolated Schiff base.

The reaction of this step preferentially gives a compound (II) in which the absolute conformation around the asymmetric carbon bonded to $R^4$ is the same as that around the asymmetric carbon bonded to $R^5$ and

$R^5$ in the compound (IV) used as reactant. To be more specific, the reaction preferentially gives R, R-isomer or S, S-isomer.

The final compound (II) of this step, after being isolated or without being isolated, is submitted to the next step in the form of a free base or an acid addition salt.

Step 1 can be further explained by way of the following example:

A mixture of compound III and the appropriate compound IV, platinum oxide and methanol can be subjected to catalytic reduction at 50 to 52°C under normal pressure for 20 hours in a hydrogen atmosphere (R/S ratio in the reaction solution: 85/15). At the end of reaction, the platinum oxide can be filtered off, the filtrate acidified by addition of an ethanolic solution of hydrogen chloride, and the solvent distilled off under reduced pressure. Acetone can be added to the residue to effect crystallization, the resulting mixture can be heated under reflux for one hour, to give a crystalline hydrochloride salt of the corresponding compound II which can be collected after cooling. The optical purity of these crystals as R,R-isomer can be improved by adding acetone, heating the resulting suspension under reflux for two hours, and collecting the crystals by filtration after cooling. This cycle of operations can be repeated four times, to afford crystals with an optical purity of 98.0%.

Still further purification can be achieved by mixing the crystals with water and acetone with heating and then alowing the mixture to cool as above.

Step 2

A compound (II) obtained in Step 1 is converted to the corresponding compound (I) or a salt thereof by reaction with tert-butyl hypochlorite followed by hydrolysis.

Compound (II) and tert-butyl hypochlorite ( in nearly equimolar or slight excess amount) are added to an organic solvent ( e.g., methanol, ethanol, ether or benzene ) under cooling. This is followed by addition of base such as an alkali metal alcoholate (e.g., sodium ethoxide). This reaction is conducted under anhydrous conditions.

After removal of the solvent, the reaction product is decarboxylated as required, and brought into contact with water in the presence of acid ( e.g., hydrochloric or sulfuric acid) preferably at room temperature.

The absolute conformation around the asymmetric carbon in the compound (I) thus obtained is identical to that around the asymmetric carbon bonded to $R^4$ in the compound (II), whether the two asymmetric carbon atoms in the compound (II) have same or different absolute conformation.

The compounds prepared by the method described above can be isolated and purified by commonly used chemical operations, such as filtration, crystallization and recrystallization.

Step 3

Step 3 converts the product from Step 2 into a free base. Examples of two methods which can be used to carry out this step are shown by the following :

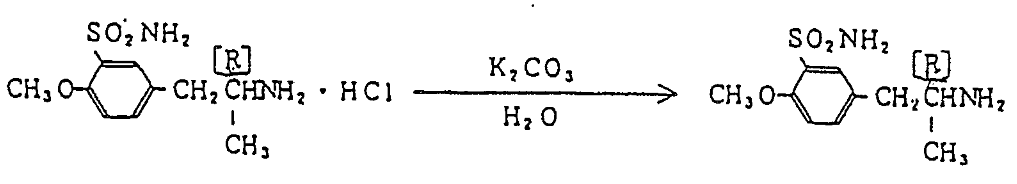


Method [A]

(R)-(-)-5-(2-aminopropyl)-2-methoxybenzenesulfonamide hydrochloride ( 20 g ) was dissolved in 140 ml water, and 50 ml of saturated potassium carbonate solution was added. After crystals separated out, the mixture was stirred at room temperature for two hours, and the crystals were collected by filtration and recrystallized from 75 ml water, affording 14.2 g of (R)-(-)-5-(2-aminopropyl)-2-methoxybenzenesulfonamide.

Yield: 82% Melting point: 166-167°C (dec.)

$[\alpha]_D^{23}$ : -17.3 ( c = 1.07, MeOH )
Elemental analysis ( $C_{10}H_{16}N_2O_3S$):

| | C(%) | H(%) | N(%) | S(%) |
|---|---|---|---|---|
| Calcd. | 49.16 | 6.60 | 11.47 | 13.12 |
| Found | 49.08 | 6.49 | 11.26 | 13.02 |

Method [B]

(R)-(-)-5-(2-aminopropyl)-2-methoxybenzenesulfonamide hydrochloride ( 4.31 g ) was dissolved in 30 ml water, 21 g anhydrous potassium carbonate was added to the solution, the mixture was stirred at room temperature for two hours, and the crystals which separated out were collected by filtration and recrystallized from 20 ml water, affording 3.14 g of (R)-(-)-5-(2-aminopropyl)-2-methoxybenzenesulfonamide. Yield: 84%.

The optically active benzenesulfonamide derivatives (I) are useful as antihypotensive agents and as intermediates for the manufacture of adrenergic receptor α-blocking agents having a side chain,

$$-CH_2\underset{\text{(lower alkyl)}}{\overset{*}{C}H}-NH-,$$

at the meta position which are described in Japanese Patent Publications No. 110665 (1981) and No. 136561 (1982).

The process of this invention is capable of producing optically active benzenesulfonamide derivatives (I) of high optical purity with higher reaction yield and optical yield, compared with the conventional method.

According to the conventional method, synthesis of one optical isomer of a compound (I) from compound (A-II) requires six reaction steps ( for hydrochloride ) or seven reaction steps ( for free base ), the overall reaction yield being about 10.5% for the former and about 9% for the latter.

In contrast, the process of this invention requires only two to four reaction steps to obtain one optical isomer of a compound (I) from a compound (III).

The overall yield is about 51.3% for hydrochloride and about 43.0% for free base under optimum conditions — 4 to 5 times higher than by the conventional method.

In addition, the process of this invention gives final products of high optical purity, and hence the optical yield is also very high [up to 99.8%].

The process of this invention is simple in operation because of the few reaction steps involved, and still higher yields can be achieved by optimum selection of reaction conditions. The reactions involved proceed very smoothly even under mild conditions and use of reactants in larger quantities does not lead to drop in yield, making the process of this invention suitable for commercial production.

Step 4

Compounds having adrenergic receptor α-blocking effect can be derived from a compound (I) according to the reaction given below,

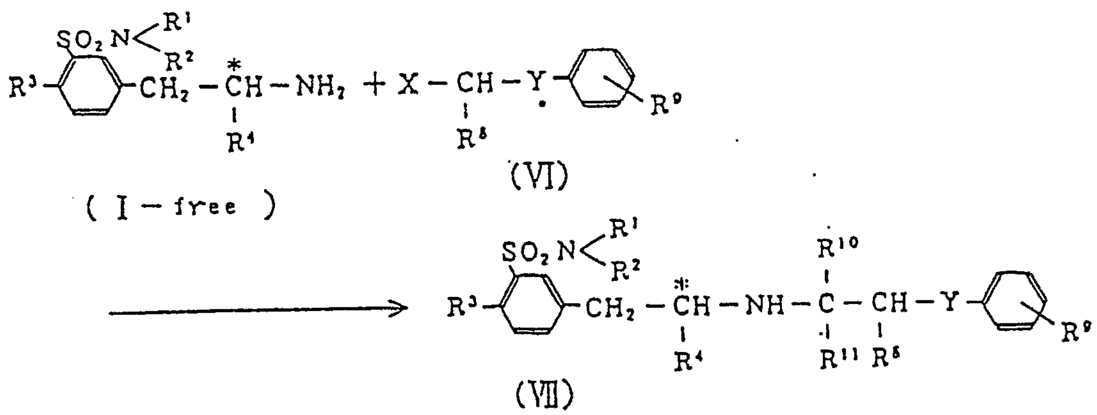

( I - free )

(VI)

(VII)

( wherein $R^1$, $R^2$, $R^3$ and $R^4$ are as defined above; X is a radical

$$\mathrm{Hal}-\overset{R^{10}}{\underset{R^{11}}{C}}-$$

or a formyl group; Hal denotes a halogen atom; $R^8$, $R^{10}$ and $R^{11}$ are selected independently from a hydrogen atom and lower alkyl groups;Y is a methylene group or oxygen atom; and $R^9$ is a hydrogen atom or a lower alkyl, lower alkoxyl or lower alkenyloxy group ).

The halogen atom Hal may be iodine, bromine or chlorine, and the lower alkenyloxy group $R^4$ is, for example, vinyloxy, allyloxy, butenyloxy, isobutenyloxy or pentenyloxy.

The optically active m-(substituted-aminoalkyl)benzenesulfonamides (VII) having adrenergic $\alpha$-blocking effect and useful as antihypertensive agents can be prepared by reaction of compound (I) ( in the form of free base ) with halide (IV), or by reductive condensation between compound (1) ( free base ) and formyl derivative of compound (VI).

It is preferable that the carbon atom to which $R^8$ is bonded and that to which $R^{10}$ and $R^{11}$ are bonded are not asymmetric carbon atoms. If either one of these is asymmetric an optically active compound (VI) previously isolated should be used for the reaction.

When X in compound (VI) is

$$\mathrm{Hal}-\overset{R^{10}}{\underset{R^{11}}{C}}-,$$

the reaction is preferably carried out in the absence of solvent or in an organic solvent ( e.g., benzene, toluene, xylene, dimethylformamide, dichloromethane, dichloroethane, methanol or ethanol ) at room temperature, at elevated temperature or under reflux, using an equal or slight molar excess amount of compound (VI).

In some instances, addition of secondary or tertiary amine ( e.g., pyridine, picoline, N,N-dimethylaniline, N-methylmorpholine, trimethylamine, triethylamine or dimethylamine ) or inorganic base ( e.g., potassium carbonate, sodium carbonate or sodium bicarbonate ) is effective in ensuring smooth reaction.

When a formyl derivative is used as compound (VI), the reaction may be carried out in much the same manner as in Step 1 of the process of this invention.

An example of the process of Step 4 is given by the following :

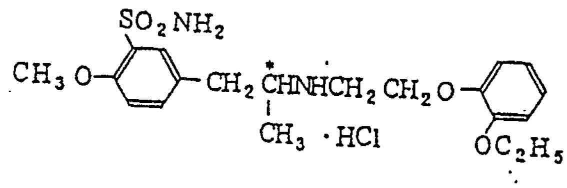

2.4 g of R(-)-5-[(2-amino-2-methyl)ethyl]-2-methoxybenzenesulfonamide (namely, (R)-(-)-5-(2-aminopropyl)-2-methoxybenzenesulfonamide) and 1.2 g of 2-(o-ethoxyphenoxy)-ethylbromide were dissolved in 120 ml of ethanol, and the mixture was refluxed for 16 hours. The solvent was distilled away, and the residue was alkalified with 10% aqueous sodium hydroxide. The separated oily material was extracted with ethyl acetate, and the extract solution was washed with saturated aqueous sodium chloride, and dried over anhydrous magnesium sulfate. The solution was subjected to distillation, and the residue was subjected to silica gel column chromatography, eluted with chloroform-methanol (9:5) to give 1.5 g of crude crystals.

The crystals were treated with ethanolic hydrochloric acid to give R(-)-5-[2-[[2-(o-ethoxyphenoxy)ethyl]-amino]-2-methylethyl]-2-methoxybenzenesulfonamide hydrochloride.

Melting point: 228-230 °C

Elemental analysis ($C_{20}H_{29}ClN_2O_5S$):

| | C(%) | H(%) | N(%) |
|---|---|---|---|
| Calcd. | 53.99 | 6.57 | 6.30 |
| Found | 53.90 | 6.64 | 6.27 |

$[\alpha]_D^{24}$ : -4.0° (c = 0.35, methanol)

## Claims

**1.** A process for producing optically active benzenesulfonamide derivative (I)

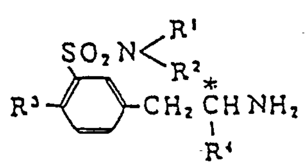

(I)

which comprises decomposing by a reaction with tert-butyl hypochlorite followed by hydrolysis m-(2-substituted-alkylaminoalkyl)benzenesulfonsmide derivative (II)

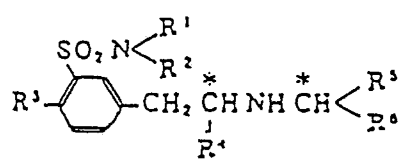

(II)

wherein $R^1$ and $R^2$ are selected independently from a hydrogen atom and $C_1$-$C_5$ all groups; $R^3$ is a hydrogen atom or a $C_1$-$C_5$ alkyl, hydroxyl or $C_1$-$C_5$ alkoxyl group; $R^4$ is a $C_1$-$C_5$ all group; $R^5$ is a $C_1$-$C_5$ alkyl, carboxy-($C_1$-$C_5$ alkyl) or ($C_1$-$C_5$ alkoxy)carbonyl-($C_1$-$C_5$ alkyl) group; and $R^6$ is a carboxyl or ($C_1$-$C_5$ alkoxy)carbonyl group.

2. A process according to claim 1 wherein the compound (II) is obtained by a method which comprises reacting sulfamoyl-substituted benzyl lower alkyl ketone (III)

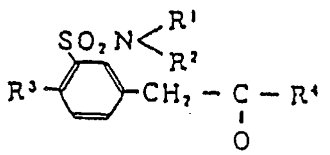

(III)

with optically active, substituted alkylamine (IV)

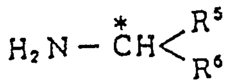

(IV)

in the presence of reducing agent.

3. A process according to claim 1 or 2 wherein product compound (I) is further reacted as follows :

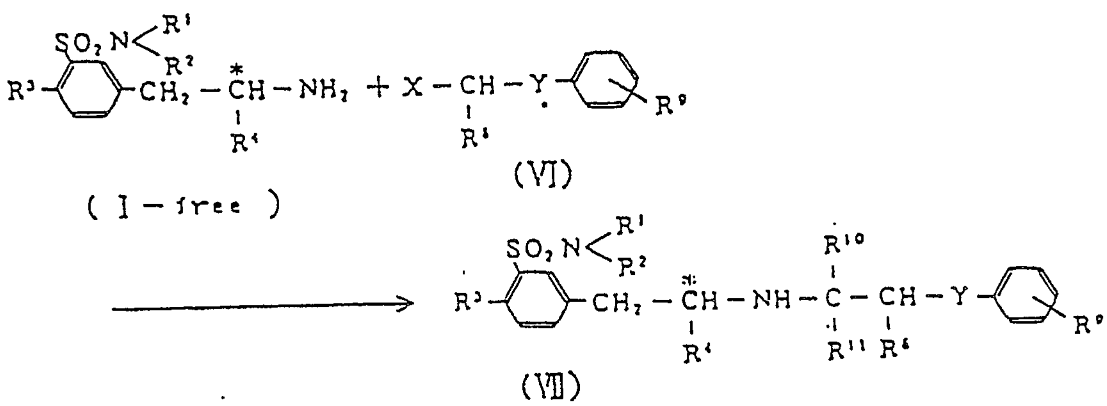

( I – free )

(VI)

(VII)

wherein X is

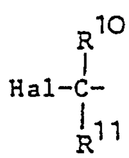

or a formyl group; Hal is a halogen atom; $R^8$, $R^{10}$ and $R^{11}$ are selected independently from a hydrogen atom and $C_1$-$C_5$ alkyl groups; Y is a methylene group or oxygen atom; and $R^9$ is a hydrogen atom or a $C_1$-$C_5$ alkyl, $C_1$-$C_5$ alkoxyl or $C_1$-$C_5$ alkenyloxy group.

4. A process according to any preceding claim wherein the decomposition is by stereospecific elimination of $R^5$-$CH_2$-$R^6$ or $R^5$-CO-$R^6$ from compound II.

5. A process according to claim 4 wherein the decomposition is effected by conversion of compound I to the form of a Schiff base followed by hydrolysis.

**Revendications**

1. Un procédé de production d'un dérivé de benzène-sulfonamide (I) optiquement actif

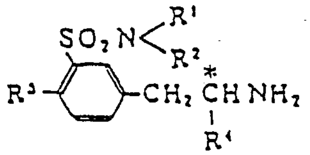

(I)

qui comprend la décomposition par une réaction avec un hypochlorite de tert-butyle, suivie par une hydrolyse, d'un dérivé de m-(2-substitué-alkylaminoalkyl) benzène-sulfonamide (II)

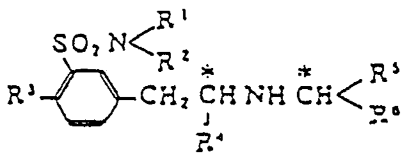

(II)

dans lequel $R^1$ et $R^2$ sont sélectionnés indépendamment parmi un atome d'hydrogène et les groupes alkyle en $C_1$ à $C_5$ ; $R^3$ est un atome d'hydrogène ou un groupe alkyle en $C_1$ à $C_5$, un groupement hydroxyle ou un groupe alcoxyle en $C_1$ à $C_5$ ; $R^4$ est un groupe alkyle en $C_1$ à $C_5$ ; $R^5$ est un groupe alkyle en $C_1$ à $C^5$, carboxy-(alkyle en $C_1$ à $C_5$) ou (alkoxy en $C_1$ à $C_5$) carbonyl-(alkyle en $C_1$ à $C_5$); et $R^6$ est un groupe carboxyle ou (alkoxy en $C_1$ à $C_5$) carbonyle.

2. Un procédé selon la revendication 1, dans lequel le composé (II) est obtenu par une méthode qui comprend la réaction d'une alkylcétone inférieure de sulfamoyl-benzyl substitué (III).

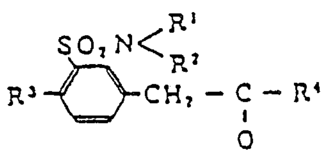

(III)

avec une alkylamine substituée (IV) optiquement active

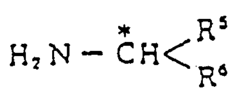

(IV)

en présence d'un agent réducteur.

3. Un procédé selon la revendication 1 ou 2, dans lequel le composé (I) de produit est en outre soumis à une réaction comme suit :

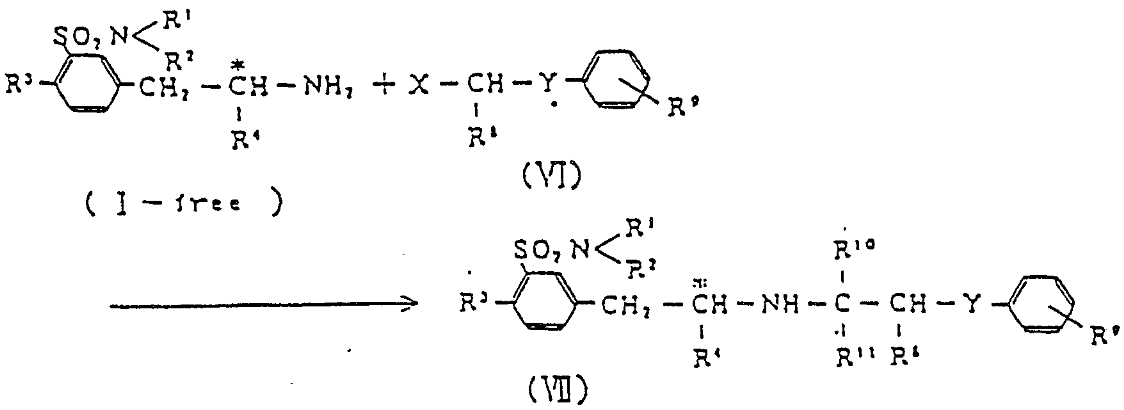

( I − free )

(VI)

(VII)

dans laquelle X est Hal-C ou un groupe formyl; Hal est un atome d'halogène ; $R^8$, $R^{10}$ et $R^{11}$ sont sélectionnés indépendamment parmi un atome d'hydrogène et les groupes alkyle en $C_1$ à $C_5$ ; Y est un groupe méthylène ou un atome d'oxygène ; et $R^9$ est un atome d'hydrogène ou un groupe alkyle en $C_1$ à $C_5$, alkoxyle en $C_1$ à $C_5$, ou alkenyloxy en $C_1$ à $C_5$.

4. Un procédé selon toute revendication précédente,
dans lequel la décomposition est faite par élimination stéréospécifique de $R^5$-$CH_2$-$R^6$ ou $R^5$-CO-$R^6$ du composé (II).

5. Procédé selon la revendication 4,
dans lequel la décomposition est effectuée par conversion du composé (I) sous la forme d'une base de Schiff, suivie d'une hydrolyse.

## Ansprüche

1. Verfahren zur Herstellung von aktivem Benzolsulfonamid-Derivat (I)

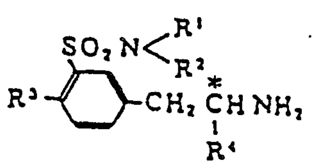

(I)

umfassend den Abbau durch eine Reaktion mit tert.Butylhypochlorit und anschließende Hydrolyse von m-(2-substituiertem Alkylaminoalkyl)benzolsulfonamid-Derivat (II)

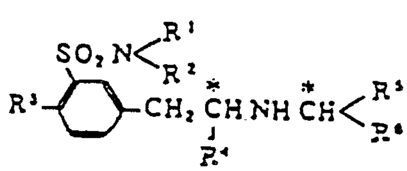

(II)

wobei $R^1$ und $R^2$ unabhängig voneinander ausgewählt sind aus einem Wasserstoffatom und $C_1$-$C_5$-Alkylgruppen; $R^3$ ein Wasserstoffatom oder eine $C_1$-$C_5$-Alkyl-, Hydroxy- oder $C_1$-$C_5$-Alkoxy-gruppe ist; $R^4$ eine $C_1$-$C_5$-Alkylgruppe ist; $R^5$ eine $C_1$-$C_5$-Alkyl-, Carboxy-($C_1$-$C_5$-Alkyl)- oder ($C_1$-$C_5$-Alkoxy)-carbonyl-($C_1$-$C_5$-alkyl)gruppe bedeutet und $R^6$ eine Carboxyl- oder ($C_1$-$C_5$-Alkoxy)carbonylgruppe ist.

2. Verfahren nach Anspruch 1, wobei die Verbindung (II) erhalten worden ist nach einem Verfahren umfassend die Umsetzung von Sulfamoyl-substituiertem Benzyl-nieder-alkylketon (III)

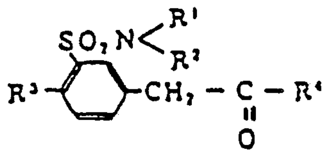

(III)

mit optisch aktivem, substituiertem Alkylamin (IV)

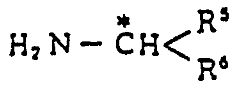

(IV)

in Gegenwart von Reduktionsmittel.

3. Verfahren nach Anspruch 1 oder 2, wobei die Produktverbindung (I) weiter wie folgt umgesetzt wird:

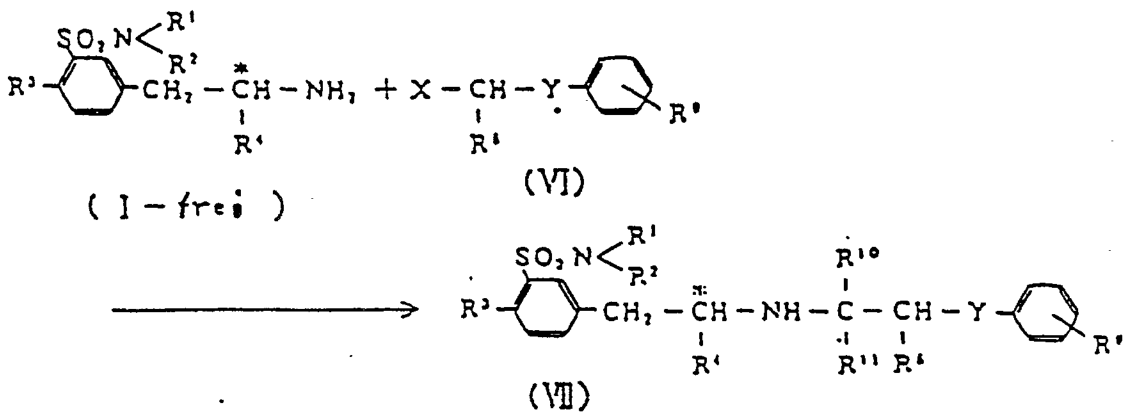

(I - frei)

(VI)

(VII)

wobei X =

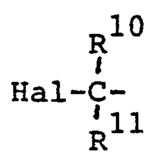

oder eine Formylgruppe ist und Hal ein Halogenatom bedeutet; $R^8$ . $R^{10}$ und $R^{11}$ unabhängig voneinander ausgewählt sind aus einem Wasserstoffatom und $C_1$-$C_5$-Alkylgruppen; Y eine Methylengruppe oder ein Sauerstoffatom ist und $R^9$ ein Wasserstoffatom oder eine $C_1$-$C_5$-Alkyl-, $C_1$-$C_5$-Alkoxy- oder $C_1$-$C_5$-Alkenyloxygruppe bedeutet.

4. Verfahren nach einem der vorangehenden Ansprüche, wobei der Abbau durchgeführt wird durch stereospezifische Abspaltung von $R^5$ -$CH_2$-$R^6$ oder $R^5$ -CO-$R^6$ von der Verbindung II.

5. Verfahren nach Anspruch 4, wobei der Abbau durchgeführt wird durch Umwandlung der Verbindung I in die Form einer Schiff'schen Base und anschließende Hydrolyse.